(19) Europäisches Patentamt
European Patent Office
Office européen des brevets

(11) Veröffentlichungsnummer: **0 007 100**
**A2**

(12) # EUROPÄISCHE PATENTANMELDUNG

(21) Anmeldenummer: **79102406.0**

(22) Anmeldetag: **12.07.79**

(51) Int. Cl.³: **C 07 C 31/18**
C 07 C 29/14, C 07 C 29/136
C 08 G 18/32, C 08 G 18/40
C 08 G 63/48

(30) Priorität: **19.07.78 DE 2831656**

(43) Veröffentlichungstag der Anmeldung:
**23.01.80 Patentblatt 80/2**

(84) Benannte Vertragsstaaten:
**BE DE FR GB IT NL**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Möhring, Edgar, Dr.**
**Hufer Weg 49a**
**D-5060 Bergisch-Gladbach 2(DE)**

(72) Erfinder: **Müller, Hanns Peter, Dr.**
**St. Pankratius Strasse 2**
**D-5068 Odenthal(DE)**

(72) Erfinder: **Wagner, Kuno, Dr.**
**Am Kiesberg 8**
**D-5090 Leverkusen 1(DE)**

(54) **Verfahren zur Herstellung von niedermolekularen Polyhydroxylverbindungen und ihre Verwendung.**

(57) Die vorliegende Patentanmeldung beansprucht ein verbessertes Verfahren zur Herstellung von niedermolekularen Polyalkoholen durch alkalische zweistufige Hydrierung eines Gemisches von niedermolekularen Hydroxyaldehyden, Hydroxyketonen und mehrwertigen Alkoholen, wie es bei der Selbstkondensation von Formaldehyd entsteht. Diese Hydrierung in Gegenwart von Metallkatalysatoren wird in zwei Stufen bei einem pH-Wert von jeweils 7,5 bis 12,5 so durchgeführt, daß der Gehalt an reduzierbaren Gruppen im Reaktor den Wert von 2 Gew.-% nicht überschreitet und daß die Lösung nach dem ersten Hydrierschritt auf einen pH-Wert von 1 bis 6 eingestellt wird und danach für den zweiten Hydrierschritt wieder alkalisch eingestellt wird. Die Patentanmeldung beansprucht weiter die Verwendung der erfindungsgemäßen Verfahrensproduckte für die Herstellung von Polyurethankunststoffen, als Startkomponente für Polyäther- und Polyesterpolyolen und für nichtionogene Tenside.

EP 0 007 100 A2

BAYER AKTIENGESELLSCHAFT
Zentralbereich
Patente, Marken und Lizenzen

5090 Leverkusen, Bayerwerk
Sft/AB-lz

# Verfahren zur Herstellung von niedermolekularen Polyhydroxylverbindungen und ihre Verwendung

Die vorliegende Erfindung betrifft ein verbessertes Verfahren zur Herstellung von niedermolekularen Polyalkoholen durch alkalische zweistufige katalytische Hydrierung eines Gemisches verschiedener niedermolekularer Hydroxyaldehyde, Hydroxyketone und gegebenenfalls mehrwertiger Alkohole, wie es bei der Selbstkondensation von Formaldehyd entsteht (im folgenden wird ein derartiges Gemisch als "Formose" bezeichnet). Die Erfindung betrifft auch die Verwendung dieser Polyalkohole für die Herstellung von Polyurethankunststoffen, Polyäther- und Polyesterpolyolen, sowie nichtionogenen Tensiden.

Seit den Arbeiten von Butlerow und Loew (Ann. 120, 295 (1861) und J. prakt. Chem. 33, 321 (1886) im vorigen Jahrhundert ist es bekannt, daß sich bei der Selbstkondensation des Formaldehydhydrats (Formosesynthese) unter dem Einfluß basischer Verbindungen, wie z.B. Calcium- oder Bleihydroxid, Hydroxyaldehyde und Hydroxyketone

bilden. In der Folge wurde die Formosesynthese immer wieder bearbeitet. Beispielsweise seien in diesem Zusammenhang Pfeil, Chem. Berichte 84, 229 (1951), Pfeil und Schroth, Chemische Berichte 85, 303 (1952), R.D. Partridge und A.H. Weiss, Carbohydrate Research 24 29-44 (1972), die Formosen aus Glycerinaldehyd bzw. Dioxyaceton nach Emil Fischer, die deutschen Patentschriften 822 385, 830 951 und 884 791, die US-Patentschriften 2 121 981, 2 224 910, 2269 935 und 2 372 378 sowie die englische Patentschrift 513 708 genannt. Diese bekannten Verfahren des Standes der Technik sind mit gewissen Nachteilen behaftet (schlechte Raum/Zeit-Ausbeuten, gefärbte Nebenprodukte); in jüngster Zeit wurden jedoch von der Anmelderin neue Verfahren entwickelt, nach denen sich in hoher Ausbeute mit gängigen Katalysatoren praktisch farblose und von störenden Nebenprodukten freie Formosen herstellen lassen.

Eines dieser neuen Verfahren besteht darin, daß man die Kondensation des Formaldehydhydrats in Gegenwart von löslichen oder unlöslichen Blei(II)salzen, bzw. an hochmolekulare Träger gebundenen Blei(II)ionen als Katalysator und von Formose als Co-Katalysator ablaufen läßt.

Die Reaktionstemperatur liegt dabei im allgemeinen zwischen 70 und 110°C, bevorzugt zwischen 80 und 100°C, und der pH-Wert der Reaktionslösung wird durch kontrollierte Zugabe einer anorganischen und/oder organischen Base bis zu einem Umsatz von 10-60 %, vorzugsweise 30-50 %, auf einen Wert von 6,0-8,0, bevorzugt 6,5-7,0, und anschließend auf einen Wert von 4,0-6,0, bevorzugt 5,0-6,0, ein-

gestellt. Überraschenderweise läßt sich die Produktverteilung der entsprechenden Polyol-, Hydroxyaldehyd- und Hydroxyketongemische durch diese spezielle pH-Führung und durch anschließende Kühlung bei verschieden hohen Restformaldehydgehalten (0 bis 10 Gew.-%, vorzugsweise 0,5 bis 6,0 Gew.-%) in reproduzierbarer Weise variieren.

Nachdem man die Selbstkondensation des Formaldehydhydrates durch Kühlen und/oder durch Desaktivierung des bleihaltigen Katalysators mittels Säuren unterbrochen hat, wird der Katalysator und gegebenenfalls auch das in den Produkten enthaltene Wasser entfernt. Hinsichtlich näherer Einzelheiten sei auf DE-OS 2 639 084 und DE-OS 2 732 077 verwiesen.

Eine weitere Möglichkeit, in hoher Raum-Zeit-Ausbeute hochkonzentrierte farblose Formosen herzustellen, besteht gemäß DE-OS 2 714 084 darin, wäßrige Formalinlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxylgruppen aufweisenden Alkohols mit einem Molekulargewicht zwischen 62 und 242 bzw. eines Gemisches derartiger Alkohole dargestellten Co-Katalysatos zu kondensieren, wobei man den pH-Wert der Reaktionslösung durch gesteuerte Zufuhr einer Base bis zu einem Umsatz von 5 bis 40 % zwischen 6,0 und 9,0 hält und anschließend bis zum Abbruch der Kondensationsreaktion auf 4,5 bis 8,0 einstellt, so daß er nun um 1,0 bis 2,0 Einheiten tiefer liegt als in der ersten Reaktionsphase, dann die

Reaktion bei einem Restgehalt von 0-10 Gew.-% Formaldehyd durch Desaktivierung des Katalysators unterbricht und den Katalysator entfernt.

Qualitativ hochwertige Formosen können auch durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohole und/oder höhermolekularer Polyhydroxylverbindungen hergestellt werden (siehe DE-OS 2 714 104).

Besonders wirtschaftlich ist es, gemäß DE-OS 2 721 093 Formose direkt, d.h. ohne den Umweg über wäßrige Formalinlösungen oder Paraformaldehyd, aus Formaldehyd enthaltenden Synthesegasen herzustellen.

Man leitet zu diesem Zweck die Synthesegase, wie sie bei der großtechnischen Herstellung von Formaldehyd anfallen, bei Temperaturen zwischen 10 und 150°C kontinuierlich oder diskontinuierlich in eine Absorptionsflüssigkeit, welche aus Wasser, ein- oder mehrwertigen niedermolekularen Alkoholen und/oder höhermolekularen Polyhydroxylverbindungen und/oder zur Endiolbildung befähigten Verbindungen als Co-Katalysator und/oder löslichen oder unlöslichen Metallverbindungen, welche gegebenenfalls an hochmolekulare Träger gebunden sind, als Katalysator besteht und einen pH-Wert von 3 bis 10 aufweist, kondensiert den Formaldehyd direkt in situ in der Absorptionsflüssigkeit (gegebenenfalls auch in einem nachgeschalteten Reaktionsrohr bzw. einer nachgeschalteten Rührkesselkaskade), bricht die Selbstkondensation des Formaldehyds bei einem Restformaldehydgehalt im Reaktionsgemisch von 0 bis 10 Gew.-% durch Kühlen und/oder durch Desaktivierung des Katalysators mittels Säuren ab und entfernt schließlich den Katalysator.

Gemische aus Hydroxyaldehyden, Hydroxyketonen und gegebenenfalls Polyalkoholen, wie sie nach den vorstehend beschriebenen Verfahren oder nach Verfahren des Standes der Technik erhalten werden, müssen für eine Reihe von Verwendungszwecken durch Reduktion der Carbonylgruppen in Gemische von Polyalkoholen umgewandelt werden (derartige, durch Reduktion von Formosen erhaltene Polyolgemische werden im folgenden "Formite" genannt). So gelingt z.B. die Reduktion von Formose direkt aus wäßriger Lösung schon bei Raumtemperatur mit Natriumborhydrid (vgl. R.D. Partridge, A.H. Weiss und D. Todd, Carbohydrate Research 24 (1972), 42); sie kann aber z.B. auch auf elektrochemischem Weg erfolgen.

Verfahren zur katalytischen Hydrierung von Zuckern, wie auch von Formose sind in großer Zahl bekannt. Dabei wird je nach Verfahrensweise mit sehr unterschiedlichen Mengen und Typen von Katalysatoren gearbeitet. So beschreiben L. Orthner und E. Gerisch (Biochem. Zeitung 259, 30 (1933)) ein Verfahren zur katalytischen Hydrierung von Formose, bei dem in 7 - 8 stündiger Reaktion bei 130°C unter 120 bar Wasserstoffdruck eine 4 %ige wäßrige Formoselösung mit 170 Gew.-%, bezogen auf Formose, an Raney-Nickel hydriert wird. Ein solches Verfahren ist natürlich in jeder Hinsicht wirtschaftlich unbefriedigend. Aus der US-Patentschrift 2 269 935 ist ein Verfahren bekannt geworden, nach dem eine ca. 40 Gew.-% Formose enthaltende

Lösung im sauren pH-Bereich mit 20 Gew.-% Nickelkatalysator bei 600 bis 620 bar Wasserstoffdruck und 120°C hydriert wird. Nachteilig an dieser Verfahrensvariante ist nicht nur der hohe Betriebsdruck, sondern auch der einzuhaltende niedrige pH-Wert, der zu durch Ni-Ionen grün gefärbten Produkten führt.

Aus der US-Patentschrift 2 224 910 ist ein Verfahren zur Hydrierung von Formose bekannt, bei dem eine 40 %ige Formoselösung mit 30 Gew.-% Raney-Nickel, bezogen auf Formose, bei 140 bis 210 bar $H_2$-Druck und pH 7 innerhalb von 4 Stunden hydriert wird. Auch dieses Verfahren befriedigt aufgrund des hohen Katalysatoraufwandes und der langen Reaktionszeit nicht.

Weitere Hydrierverfahren sind in den deutschen Patentschriften 705 274, 725 842, 830 951, 888 096 und 1 004 157 sowie in den US-Patentschriften 2 271 083, 2 272 378, 2 276 192, 2 760 983 und 2 775 621 beschrieben. Alle diese Verfahren weisen jedoch einen oder mehrere der folgenden Nachteile auf: großer apparativer Aufwand und schwierige Handhabung wegen hoher Wasserstoffdrucke; hoher Katalysatoraufwand, bezogen auf das hydrierte Produkt (10-200 Gew.-%); gefärbte Produkte durch lange Hydrierzeiten (1-10 Stunden).

Gemeinsam ist allen bisher bekannten Verfahren die Verwendung von Metall- oder gegebenenfalls Edelmetallkatalysatoren. Insbesondere wird Raney-Nickel eingesetzt, welches jedoch erst im alkalischen Bereich seine volle Aktivität entfaltet. Da aber andererseits im alkalischen Milieu die Formose zur Karamelisierung neigt und stark verfärbte Produkte entstehen, wird

nach den Verfahren des Standes der Technik im allgemeinen nicht im alkalischen, sondern im schwach sauren bzw. neutralen pH-Bereich gearbeitet.

Gemäß einem eigenen älteren Vorschlag (Deutsche Patentanmeldung P 27 56 270.5) ist es jedoch möglich, Formoselösungen (gegebenenfalls im Gemisch mit anderen natürlichen und/oder synthetischen Zuckern) in stark alkalischem Milieu in schneller Reaktion, mit niedrigem Katalysatoraufwand, bei Wasserstoffdrücken zwischen 100 und 200 bar und Temperaturen zwischen 50 bis 250°C zu farblosen Lösungen von Polyolgemischen zu hydrieren.

Nach dem Verfahren der Deutschen Patentanmeldung P 27 562 70.5 wird eine mehr als 20 %ige Formoselösung chargenweise so in einen Reaktor eingepumpt, der eine Temperatur von ca. 100-200°C hat, daß die Konzentration an den zu reduzierenden Gruppen 2 Gew.-% nicht übersteigt. Hydriert wird bei einem pH-Wert von 7,5 - 12,5 mit einem Katalysatoraufwand von $10^{-4}$ bis $5 \times 10^{-2}$ Gew.-%, (bezogen auf Formose) und bei einem Wasserstoffdruck von 50 - 300 bar, wobei als Katalysatoren insbesondere Metalle mit den Ordnungszahlen 23-29 (speziell Raney-Nickel) verwendet werden.

Beim Einengen der nach bisher bekannten Hydrierverfahren erhaltenen Formitlösungen, beispielswiese durch Dünnschichtdestillation im Vakuum, treten im allgemeinen störende gelbe Verfärbungen auf, deren Ursache bisher unbekannt ist. Dabei ist es unerheblich, nach welchem Verfahren die Formoselösung hergestellt und unter welchen Bedingungen sie hydriert wurde.

Überraschenderweise wurde nun gefunden, daß man das Auftreten von Verfärbungen beim Einengen der Formitlösungen vollständig unterbinden kann, wenn man bei der Hydrierung eine spezielle zweistufige Verfahrensweise anwendet:

In einer ersten Stufe wird im alkalischen Milieu die Formose, vorzugsweise an einem Raney-Metall-Kontakt, hydriert, die Lösung anschließend auf pH 1-6 eingestellt, dann wieder alkalisch gemacht und in einer zweiten Stufe im alkalischen Milieu nachhydriert.

Gegenstand der Erfindung ist somit ein Verfahren zur Herstellung von niedermolekularen, mehrwertigen Alkoholen durch Hydrierung von Formoselösungen, in Gegenwart von Metallkatalysatoren, welches dadurch gekennzeichnet ist, daß man

A) in einer ersten Stufe eine mindestens 20 %ige, bevorzugt mehr als 35 %ige, besonders bevorzugt mehr als 45 %ige Lösung von Formose, deren pH-Wert unmittelbar vor der Hydrierungsreaktion auf einen Wert im Bereich zwischen 7,5 und 12,5, vorzugsweise zwischen 8,5 und 11,5, eingestellt wurde, chargenweise in einen - vorzugsweise auf einer Temperatur von 100 bis 200°C, besonders bevorzugt 140 bis 190°C, gehaltenen - Reaktor einbringt, so daß der Gehalt an reduzierbaren Gruppen (bestimmt als Carbonylgruppen) im Produktgemisch innerhalb des Reaktors einen Wert von 2 Gew.-%, vorzugsweise 1 Gew.-%, besonders bevorzugt 0,5 Gew.-%, nicht überschreitet, und chargenweise in Gegenwart einer Gesamtmenge an Katalysator von $10^{-4}$ bis $5.10^{-2}$ Gew.-%, bezogen auf

Gesamtmenge an zu reduzierendem Ausgangsprodukt, hydriert, wobei der Katalysator stationär im Reaktor verbleibt, danach das Reaktionsprodukt chargenweise aus dem Reaktor abzieht, nachdem der Gehalt an reduzierbaren Gruppen (bestimmt als Carbonylgruppen) unter 1,5 Gew.-%, vorzugsweise unter 0,5 Gew.-%, gesunken ist, den pH-Wert des so vorhydrierten Reaktionsprodukts auf 1 bis 6, vorzugsweise 1 bis 4,5, einstellt und

B) in einer zweiten Stufe das Reaktionsprodukt der ersten Stufe chargenweise in einen - vorzugsweise auf einer Temperatur von 50 - 250°C, besonders bevorzugt 100 - 200°C, gehaltenen zweiten Reaktor einbringt, wobei der pH-Wert des Produkts unmittelbar vor dem Einpumpen in den zweiten Reaktor wieder auf einen Wert im Bereich von 7,5 bis 12,5, vorzugsweise 8,5 bis 11,5,eingestellt wird, chargenweise in Gegenwart einer Gesamtmenge an Katalysator von $10^{-4}$ bis $5\cdot10^{-2}$ Gew.-%, bezogen auf Gesamtmenge an in der ersten Stufe vorhydriertem Produkt, hydriert, wobei der Katalysator stationär im Reaktor verbleibt, und nach einer Verweilzeit von 5 min.bis 4 Stunden, bevorzugt 10 min. bis 60 min.,das Reaktionsprodukt chargenweise aus dem Reaktor abzieht.

Das erfindungsgemäße Verfahren wird besonders vorteilhaft in folgender Weise ausgeführt:

In einem Druckreaktor wird die Menge an Katalysator (bevorzugt Raney-Nickel) in Wasser vorgelegt, die zur Hydrierung des gesamten Ansatzes eingesetzt werden soll. Dann wird der Reaktor mit Wasserstoffgas bis zum Betriebsdruck von 50 bis 300 bar gefüllt und anschließend auf die

Hydriertemperatur von 80 bis 220°C aufgeheizt. Dann wird die drei- bis dreißigfache Menge (bevorzugt fünf- bis zwanzigfache Menge) Formoselösung, bezogen auf Katalysator, langsam (d.h. etwa 1/6 des Reaktorvolumens in 3 Minuten bis 2 Stunden, vorzugsweise in 5 bis 30 Minuten) zugepumpt. Man läßt danach die halbe bis die vierfache Zeit, die zum Zupumpen benötigt wurde, nachhydrieren. Anschließend drückt man die Menge Reaktionsgemisch, die der zugepumpten Menge Formoselösung entspricht, über eine Stahlmantelfritte ab, wobei der Katalysator im Reaktor verbleibt. Anschließend wird eine neue Charge in den Reaktor gepumpt und mit dieser ebenso verfahren wie mit der ersten Charge. Alle weiteren Chargen werden in gleicher Weise behandelt. Jede Charge wird - vorteilhafterweise unmittelbar anschließend -, bei einer Temperatur von 10 bis 100 °C für 10 min. bis 2 Stunden, bevorzugt 15 min. bis 1 Stunde auf pH 1 bis 6 gehalten und danach wieder auf pH 7,5 bis 12,5 eingestellt und anschließend in einen zweiten Reaktor eingebracht.

In diesem zweiten Reaktor ist ebenfalls vorzugsweise die gesamte Menge an Katalysator, welche zur Hydrierung der Gesamtmenge des in der ersten Stufe vorhydrierten Produkts vorgesehen ist, in Wasser vorgelegt. Der Reaktor wird mit Wasserstoffgas bis zu einen Betriebsdruck von 50 bis 300 bar gefüllt und anschließend auf die Hydriertemperatur von 50 - 250°C aufgeheizt.

Das Einbringen geschieht bevorzugt in der Weise, daß man die drei- bis dreißigfache (besonders bevorzugt

fünf- bis zwanzigfache) Menge an vorhydrierter Formoselösung, bezogen auf Katalysator, langsam (d.h. etwa 1/6 des Reaktorvolumens in 3 Minuten bis 2 Stunden, vorzugsweise in 5 bis 30 min.) zupumpt. Man läßt danach die halbe bis fünffache Zeit, die zum Zupumpen benötigt wurde, nachhydrieren. Anschließend drückt man die gleiche Menge Reaktionsprodukt über eine Stahlmantelfritte ab, wobei der Katalysator im Reaktor verbleibt. Dann wird die nächste, aus dem ersten Reaktor anfallende Charge nach Ansäuern und neuerlichem Alkalischmachen in den zweiten Reaktor gepumpt und mit dieser ebenso verfahren wie mit der vorangegangenen. Alle weiteren Chargen werden in gleicher Weise behandelt.

Diese erfindungsgemäße, zweistufige, chargenweise Pumphydrierung ermöglicht sowohl für die erste Stufe als auch für die zweite Stufe extrem hohe Katalysatorstandzeiten und somit - bezogen auf die Gesamtmenge an zu reduzierender bzw. an reduzierter Formose - einen sehr kleinen Katalysatoraufwand.

Neben den in der Deutschen Patentanmeldung P 27 56 720.5 beschriebenen Vorteilen der Pumphydrierung von Formose - verglichen mit den bekannten Verfahren des Standes der Technik - wie

1) hohe Wirtschaftlichkeit des Verfahrens infolge geringen Katalysatoraufwands und kurzer Hydrierzeiten;

2) geringer technischer Aufwand wegen relativ niedriger Wasserstoffdrücke;

3) weitgehende Spaltung der Ausgangssubstanzen in niedermolekulare $C_2$- bis $C_5$-Alkohole, wodurch die Viskosität der Polyhydroxylverbindungen gesenkt, ihre Verarbeitbarkeit verbessert und gleichzeitig die Verträglichkeit gegenüber anderen Substanzen erhöht wird, speziell mit den bei der Herstellung von Kunststoffen nach dem Polyisocyanat-Polyadditionsverfahren verwendeten Ausgangskomponenten (insbesondere höhermolekulare Polyhydroxylverbindungen und Treibmittel);

4) Möglichkeit, gewünschtenfalls weitere Verbindungen wie z.B. Alkanale, ein-oder mehrwertige Alkohole, Ketone, Aldehyde oder höhermolekulare Polyole in Mengen bis zu 50 Gew.-% (bezogen auf Gesamtmenge der zu reduzierenden Produkte) der zu hydrierenden Formose zuzusetzen, da diese insbesondere die Verträglichkeit der Reaktionsprodukte gegenüber den beim Polyisocyanat-Polyadditionsverfahren verwendeten Treibmitteln verbessern;

5) Möglichkeit, nach dem erfindungsgemäßen Verfahren neben Formose auch andere natürliche und/oder künstliche Zucker zu hydrieren;

welche alle auf das erfindungsgemäße zweistufige Pumphydrierverfahren ebenfalls zutreffen, bietet das erfindungsgemäße Verfahren den wesentlichen Vorteil, farblose Polyolgemischlösungen zu liefern, die ohne jegliche Verfärbung bei Temperaturen bis zu 180°C eingeengt werden können.

Dies ist von besonderem Vorteil, da für eine Reihe von Anwendungsgebieten die durch Hydrierung von Formose

erhältlichen Polyhydroxylverbindungen erst nach Entfernung des als Lösungsmittel dienenden Wassers eingesetzt werden können, wobei aber eine Verfärbung prohibitiv sein kann (z.B. bei der Verwendung als Polyolkomponente in Polyurethanlacken).

Als Aldehyde bzw. Alkanale, die im erfindungsgemäßen Verfahren gegebenenfalls mitverwendet werden können, kommen insbesondere Acetaldehyd, Propionaldehyd, Butyraldehyd, Isobutyraldehyd sowie deren Methylolderivate in Frage.

Als Ketone seien Aceton, Methyläthylketon, Diäthylketon, Cyclopentanon, Cyclohexanon, Mesityloxid, Isophoron, Acetophenon, Benzophenon sowie deren Methylolderivate genannt.

Als Lösungsmittel kommt im erfindungsgemäßen Verfahren in erster Linie Wasser in Frage; die Formose kann jedoch auch in beliebigen Mono- oder Polyalkoholen gelöst sein. Geeignete Alkohole sind z.B. Methanol, Äthanol, Propanol, Butanol, Isopropanol, Isobutanol, Cyclopentanol, Cyclohexanol, 2-Äthoxy-äthanol, 2-Propoxyäthanol, 2-Isopropoxyäthanol, 2-Butoxyäthanol, 2-(2-Methoxyäthoxy)-äthanol, 2-(2-Äthoxyäthoxy)-äthanol, 1,2-Bis(2-hydroxyäthoxy)-äthan, Äthylenglykol, Diäthylenglykol, Triäthylenglykol, Tetraäthylenglykol, 1,2-Propandiol, Isopropylenglykol, 1,3-Propandiol, 1,2-Butandiol, 1,3-Butandiol, 2-Methoxy-1-butanol, 2,3-Butandiol, 1.5-Pentandiol, 2,2-Dimethyl-1,3-propandiol, 1,6-Hexandiol, 2,5-Hexandiol, 2-Methyl-2,4-pentandiol, 3-Methyl-1,5-pentandiol, 3-Methyl-2,4-pentandiol, 2,3-Dimethyl-2,3-butandiol, 2-Methyl-2-propyl-1,3-propandiol, 2,2-Diäthyl-1,3-propandiol, 2-Äthyl-1,3-hexandiol, 2,5-Dimethyl-2,5-hexandiol, 2,2,4-Trimethyl-1,3-pentandiol, 1,3-Diäthoxy-2-propanol, 2-Hydroxymethyl-2-methyl-1,3-propandiol, 1,2,6-Hexantriol, 2-Äthyl-2-hydroxymethyl-1,3-propandiol, 2,2-Bis-hydroxymethyl-1,3-propandiol, Erythrit, Chinit, Mannit, Sorbit und Methylglykosid, sowie Äthoxylierungs- und Propoxylierungsprodukte dieser Alkohole mit einem Molekulargewicht bis ca. 400 und selbstverständlich auch Gemische dieser Alkohole. Besonders bevorzugt sind Äthylenglykol, Glycerin und 1,4-Butandiol.

Erfindungsgemäß können bei der Formosehydrierung - gegebenenfalls im Gemisch mit den obengenannten Alkoholen - jedoch auch Polyhydroxylverbindungen mit einem Molekulargewicht von 400 bis 10 000, vorzugsweise 500 bis 6000, mitverwendet werden, welche zweckmäßigerweise ebenfalls bei Raumtemperatur flüssig bzw. in der Formoselösung löslich sind, z.B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind.

Das erfindungsgemäße Hydrierverfahren ist auf beliebige Formosen anwendbar, wie sie bei den Verfahren des eingangs zitierten Standes der Technik erhalten werden. Die Formose kann jedoch auch im Gemisch mit bis zu 80 Gew.-% (bezogen auf Gesamtmenge an zu hydrierenden Verbindungen) an anderen künstlichen oder auch natürlichen Zuckern (wie z.B. Glucose, Maltose, Fructose,

Saccharose, Lactose etc.) eingesetzt werden. Es ist dabei von Vorteil, daß Formose ein ausgezeichnetes Lösungsmittel bzw. einen Löslichkeitsvermittler für derartige Zucker darstellt.

Als erfindungsgemäß mitverwendbare künstliche Invertzucker kommen Hydrolysate von beliebigen Di- und/oder Polysacchariden in Frage, z.B. von Rohrzucker, Mischungen von Rohrzucker und Invertzuckern, Hydrolysate von Trehalose, Maltose oder Isomaltose, Hydrolysate von Mais- und Kartoffelstärke und von Pektinstoffen (Amylose und Amylopektine), Cellobiose und Lactose (Milchzucker), Hydrolysate von Galaktose, Glukosemischungen, Raffinose-Hydrolysate, Cellulose-Hydrolysate, Hydrolysate von Dextrinen, gegebenenfalls in Mischung mit nichthydrolysierten Dextrinen, Hydrolysate der Schardinger-Dextrine (cyclische Dextrine), Hydrolysate des Glykogens, Hydrolysate der Glukose-6-Phosphorsäure, Hydrolysate von Glukose-1-Phosphat (Cori-Ester), Fruktose-6-Phosphat, abgebaute Pektinstoffe (Polygalakturonsäuren), abgebaute Glukosamine, Hydrolysate von Melasserückständen etc.

Der pH-Wert der zu hydrierenden Lösung kann bei beiden Stufen des erfindungsgemäßen Verfahrens sowohl mit anorganischen als auch mit organischen Basen eingestellt werden. Bevorzugt sind Natriumhydroxid, Kaliumhydroxid, Calciumhydroxid, Magnesiumhydroxid, Bariumhydroxid, Aluminiumoxidhydrat, Triäthylamin, N-Methylmorpholin und N-Methylpiperidin.

Besonders bevorzugt ist jeweils die bei der Formosesynthese verwendete Base, die vor der Hydrierung durch Behandeln der Formoselösung mit in der OH-Form vorliegendem Ionenaustauscherharz in die freie OH-Form übergeführt wird, wodurch sich die benötigte Alkalität der Formoselösung von selbst einstellt.

Für das Ansäuern zwischen der ersten und der zweiten Stufe des erfindungsgemäßen Verfahrens eignen sich Mineralsäuren wie z.B. Salzsäure, Salpetersäure oder Phosphorsäure, organische Säuren wie Oxalsäure, Ameisensäure oder Essigsäure und bevorzugt in der $H^{\oplus}$ Form vorliegende stark saure Kationenaustauscher.

Als Hydrier-Katalysatoren kommen für das erfindungsgemäße Verfahren insbesondere Metalle mit den Ordnungszahlen 23 bis 29 (in elementarer und/oder oxidischer Form) in Frage. Geeignete Katalysatoren sind beispielsweise solche auf Basis von Nickel oder Kobalt, wobei als Träger für den Katalysator sowohl anorganische Materialien wie Kieselgur, Kieselsäuren, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Eisenoxid, Zinkoxid, Calciumcarbonat, Siliziumcarbid, Aluminiumphosphat, Borphosphat, Asbest oder Aktiv-Kohle als auch organische Materialien, z.B. natürlich vorkommende oder synthetische Verbindungen mit hohem Molgewicht wie Seide, Polyamide, Polystyrole, Zellstoff oder Polyurethane verwendbar sind; das Trägermaterial kann dabei z.B. in Form von Kugeln, Strängen, Fäden, Zylindern, Polygonen oder in Pulverform vorliegen. Bevorzugt sind Raney-Typ-Katalysatoren, wie Raney-Nickel, W-1-, W-5-, W-6- und W-7-Raney-Nickel (siehe H. Adkins, J.Am.Chem. Soc. 69, 3039 (1974)), Raney-Kobalt-Katalysatoren, Raney-Kupfer, Raney-Nickel-Eisen, Raney-Kobalt-Nickel und Raney-Kobalt-Eisen. In Betracht kommen auch durch Reduktion von Nickel- oder Kobaltsalzen hergestellte

Metallkatalysatoren, wie Urushibara-Nickel oder mit Metallalkylverbindungen, Alkalihydriden, Hydrazin, Boranaten oder Borwasserstoff reduzierte Nickel- oder Kobaltsalze, durch Reduktion der Metalloxide oder Metalloxidgemische hergestellte Katalysatoren, aber auch die Metalloxide oder -oxidgemische selbst.

Die erfindungsgemäß bevorzugten Katalysatoren auf Basis der Metalle mit den Ordnungszahlen 23 - 29 können als Beschleuniger eines oder mehrere der folgenden Elemente in Mengen bis zu 10 Gew.-% enthalten:

Li, Na, Ca, Ba, K, Ag, Be, La, Ce, V, Nb, Ta, Mo, W, und bis zu 1 Gew.-% der Elemente Ru, Rh, Pd, Au, Ir, Pt.

Besonders geeignete Katalysatoren sind Raney-Nickel mit 90 Gew.-% Ni und < 1 Gew.-% Fe, Ca und Na, Raney-Nickel-Eisen mit 5 bis 30 Gew.-% Fe und < 1 Gew.-% Ca und Na und Raney-Kobalt-Eisen mit 10 - 30 Gew.-% Fe.

Bevorzugter Verwendungszweck der erfindungsgemäß hergestellten Gemische von mehrwertigen, niedermolekularen Alkoholen ist ihr Einsatz als Polyolkomponente im Polyisocyanat-Polyadditionsverfahren.

Gegenstand der vorliegenden Erfindung ist somit auch ein Verfahren zur Herstellung von gegebenenfalls zellförmigen Polyurethankunststoffen durch Umsetzung von

A) Polyisocyanaten mit
B) mindestens zwei aktive Wasserstoffatome aufweisenden Verbindungen mit einem Molekulargewicht zwischen 32 und 400, gegebenenfalls

C) mindestens zwei aktive Wasserstoffatome aufweisenden Verbindungen mit einem Molekulargewicht zwischen 400 und 10 000 sowie gegebenenfalls

D) Treibmitteln, Katalysatoren und weiteren an sich bekannten Zusatzstoffen,

welches dadurch gekennzeichnet ist, daß als Komponente B), gegebenenfalls nur anteilsweise, erfindungsgemäß hergestellte Gemische niedermolekularer, mehrwertiger Alkohole eingesetzt werden.

Für die Durchführung des erfindungsgemäßen Verfahrens werden eingesetzt:

1. Als Ausgangskomponenten aliphatische, cycloaliphatische, araliphatische, aromatische und heterocyclische Polyisocyanate, wie sie z.B. von W. Siefken in Justus Liebigs Annalen der Chemie, 562, Seiten 75 bis 136, beschrieben werden, beispielsweise solche der Formel

$$Q\ (NCO)_n$$

in der
$n$ = 2-4, vorzugsweise 2,
und
Q einen aliphatischen Kohlenwasserstoffrest mit 2-18, vorzugsweise 6-10 C-Atomen,
einen cycloaliphatischen Kohlenwasserstoffrest mit 4-15, vorzugsweise 5-10 C-Atomen,
einen aromatischen Kohlenwasserstoffrest mit 6-15, vorzugsweise 6-13 C-Atomen,
oder einen araliphatischen Kohlenwasserstoffrest mit 8-15, vorzugsweise 8 - 13 C-Atomen,

bedeuten, z.B. Äthylen-diisocyanat, 1,4-Tetramethylen-diisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'-und/oder -4,4'-diisocyanat, sowie Naphthylen-1,5-diisocyanat.

Ferner kommen beispielsweise erfindungsgemäß in Frage: Triphenylmethan-4,4',4"-triisocyanat, Polyphenyl-polymethylen-polyisocyanate, wie sie durch Anilin-Formaldehyd-Kondensation und anschließende Phosgenierung erhalten und z.B. in den GB-Patentschriften 874 430 und 848 671 beschrieben werden, m- und p-Isocyanatophenylsulfonyl-isocyanate gemäß der US-Patentschrift 3 454 606, perchlorierte Arylpolyisocyanate, wie sie z.B. in der DE-Auslegeschrift 1 157 601 (US-Patentschrift 3 277 138) beschrieben werden, Carbodiimidgruppen aufweisende Polyisocyanate, wie sie in der DE-Patentschrift 1 092 007 (US-Patentschrift 3 152 162) sowie in den DE-Offenlegungsschriften 2 504 400, 2 537 685 und 2 552 350 beschrieben werden, Norbornan-Diisocyanate gemäß US-Patentschrift 3 492 330, Allophanatgruppen aufweisende Polyisocyanate, wie sie z.B. in der GB-Patentschrift 994 890, der BE-Patentschrift 761 626 und der NL-Patentanmeldung 7 102 524 beschrieben werden, Isocyanuratgruppen aufweisende Polyisocyanate, wie sie z.B. in der US-Patentschrift 3 001 973, in den DE-Patentschriften 1 022 789, 1 222 067 und

1 027 394 sowie in den DE-Offenlegungsschriften 1 929 034 und 2 004 048 beschrieben werden, Urethangruppen aufweisende Polyisocyanate, wie sie z.B. in der BE-Patentschrift 752 261 oder in den US-Patentschriften 3 394 164 und 3 644 457 beschrieben werden, acylierte Harnstoffgruppen aufweisende Polyisocyanate gemäß der DE-Patentschrift 1 230 778, Biuretgruppen aufweisende Polyisocyanate, wie sie z.B. in den US-Patentschriften 3 124 605, 3 201 372 und 3 124 605 sowie in der GB-Patentschrift 889 050 beschrieben werden, durch Telomerisationsreaktionen hergestellte Polyisocyanate, wie sie z.B. in der US-Patentschrift 3 654 106 beschrieben werden, Estergruppen aufweisende Polyisocyanate, wie sie z.B. in den GB-Patentschriften 965 474 und 1 072 956, in der US-Patentschrift 3 567 763 und in der DE-Patentschrift 1 231 688 genannt werden, Umsetzungsprodukte der obengenannten Isocyanate mit Acetalen gemäß der DE-Patentschrift 1 072 385 und polymere Fettsäureester enthaltende Polyisocyanate gemäß der US-Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten.

2. Als Ausgangskomponenten ferner gegebenenfalls Verbindungen mit mindestens zwei gegenüber Isocyanaten reaktionsfähigen Wasserstoffatomen von einem Molekulargewicht in der Regel von 400 - 10 000. Hierunter versteht man neben Aminogruppen, Thiolgruppen oder Carboxylgruppen aufweisenden Verbindungen vorzugsweise Hydroxylgruppen aufweisende Verbindungen, insbesondere zwei bis acht Hydroxylgruppen aufweisende Verbindungen, speziell solche vom Molekulargewicht 500 bis 7000, vorzugsweise 1000 bis 5000, z.B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind:

a) Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele für solche Carbonsäuren und deren Derivate seien genannt:

Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit monomeren ungesättigten Fettsäuren, wie Ölsäure; Terephthalsäuredimethylester und Terephthalsäure-bisglykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol,Glycerin, Trimethylolpropan, Hexantriol-(1,2,6), Butantriol-(1,2,4), Trimethyloläthan, Pentaerythrit, Chinit, Mannit und Sorbit, Formit, Methylglykosid, ferner Diäthylenglykol, Triäthylenglykol, Tetraäthylenglykol und höhere Polyäthylenglykole, Dipropylenglykol und höhere Polypropylenglykole sowie Dibutylenglykol und höhere Polybutylenglykole in Frage. Die Polyester können anteilig endständige Carboxylgruppen aufweisen. Auch Polyester aus Lactonen, z.B. $\varepsilon$-Caprolacton, oder aus Hydroxycarbonsäuren, z.B. $\omega$-Hydroxycapronsäure, sind einsetzbar.

b) Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von Lewis-Katalysatoren wie $BF_3$, oder durch Anlagerung dieser Epoxide, vorzugsweise von Äthylenoxid und Propylenoxid , gegebenenfalls im Gemisch oder nacheinander, an Startkomponenten mit reaktionsfähigen Wasserstoffatomen wie Wasser, Alkohole, Ammoniak oder Amine, z.B. Äthylenglykol, Propylenglykol-(1,3) oder -(1,2), Trimethylolpropan, Glycerin, Sorbit, 4,4'-Dihydroxy-diphenylpropan, Anilin, Äthanolamin oder Äthylendiamin hergestellt. Auch Sucrosepolyäther, wie sie z.B. in den DE-Auslegeschriften 1 176 358 und 1 064 938 beschrieben werden, sowie auf Formit oder Formose gestartete Polyäther (DE-Offenlegungsschriften 2 639 083 bzw. 2 737 951), kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch OH-Gruppen aufweisende Polybutadiene sind erfindungsgemäß geeignet.

c) Unter den Polythioäthern seien insbesondere die Kondensationsprodukte von Thiodiglykol mit sich selbst und/oder mit anderen Glykolen, Dicarbonsäuren,

Formaldehyd, Aminocarbonsäuren oder Aminoalkoholen angeführt. Je nach den Co-Komponenten handelt es sich bei den Produkten z.B. um Polythiomischäther, Polythioätherester oder Polythioätheresteramide.

d) Als Polyacetale kommen z.B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale wie z.B. Trioxan (DE-Offenlegungsschrift 1 694 128) lassen sich erfindungsgemäß geeignete Polyacetale herstellen.

e) Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol, Tetraäthylenglykol oder Thiodiglykol mit Diarylcarbonaten, z.B. Diphenylcarbonat, oder Phosgen hergestellt werden können (DE-Auslegeschriften 1 694 080, 1 915 908 und 2 221 751; DE-Offenlegungsschrift 2 605 024).

f) Zu den Polyesteramiden und Polyamiden zählen z.B. die aus mehrwertigen gesättigten oder ungesättigten Carbonsäuren bzw. deren Anhydriden und mehrwertigen gesättigten oder ungesättigten Aminoalkoholen, Diaminen, Polyaminen und deren Mischungen gewonnenen, vorwiegend linearen Kondensate.

g) Auch bereits Urethan- oder Harnstoffgruppen enthaltende Polyhydroxylverbindungen sowie gegebenenfalls modifizierte natürliche Polyole, wie Rizinusöl oder Kohlenhydrate, z.B. Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

h) Die genannten Polyhydroxylverbindungen können vor ihrer Verwendung im Polyisocyanat-Polyadditionsverfahren noch in der verschiedensten Weise modifiziert werden: So läßt sich gemäß DE-Offenlegungsschriften 2 210 839 (US-Patentschrift 3 849 515) und 2 544 195 ein Gemisch aus verschiedenen Polyhydroxylverbindungen (z.B. aus einem Polyäther- und einem Polyesterpolyol) durch Verätherung in Gegenwart einer starken Säure zu einem höhermolekularen Polyol kondensieren, welches aus über Ätherbrücken verbundenen verschiedenen Segmenten aufgebaut ist. Es ist auch möglich, z.B. gemäß DE-Offenlegungsschrift 2 559 372 in die Polyhydroxylverbindungen Amidgruppen oder gemäß DE-Offenlegungsschrift 2 620 487 durch Umsetzung mit polyfunktionellen Cyansäureestern Triazingruppen einzuführen. Durch Umsetzung eines Polyols mit einer weniger als äquivalenten Menge eines Diisocyanatocarbodiimids und anschließende Reaktion der Carbodiimidgruppe mit einem Amin, Amid, Phosphit oder einer Carbonsäure erhält man Guanidin-, Phosphonoformamidin- bzw. Acylharnstoffgruppen aufweisende Polyhydroxylverbindungen (DE-Offenlegungsschriften 2 714 289, 2 714 292 und 2 714 293). Von besonderem Interesse ist es in manchen Fällen, die höhermolekularen Polyhydroxylverbindungen durch Reaktion mit Isatosäureanhydrid vollständig oder teilweise in die entsprechenden Anthranilsäureester überzuführen, wie es in den DE-Offenlegungsschriften 2 019 432 und 2 619 840 bzw. den US-Patentschriften 3 808 250, 3 975 428 und 4 016 143 beschrieben ist. Man erhält auf diese Weise höhermolekulare Verbindungen mit endständigen aromatischen Aminogruppen.

Durch Umsetzung von NCO-Präpolymeren mit Hydroxylgruppen aufweisenden Enaminen, Aldiminen oder Ketiminen und anschließende Hydrolyse erhält man gemäß DE-Offenlegungsschrift 2 546 536 bzw. US-Patentschrift 3 865 791 höhermolekulare, endständige Aminogruppen aufweisende Verbindungen. Weitere Herstellungsverfahren für höhermolekulare Verbindungen mit endständigen Aminogruppen oder Hydrazidgruppen werden in der DE-Offenlegungsschrift 1 694 152 (US-Patentschrift 3 625 871) beschrieben.

i) Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z.B. erhalten, wenn man Polyadditionsreaktionen (z.B. Umsetzungen zwischen Polyisocyanaten und amino-

funktionellen Verbindungen) bzw. Polykondensationsreaktionen (z.B. zwischen Formaldehyd und Phenolen und/oder Aminen) in situ in den oben genannten, Hydroxylgruppen aufweisenden Verbindungen ablaufen läßt. Derartige Verfahren sind beispielsweise in den DE-Auslegeschriften 1 168 075 und 1 260 142, sowie den DE-Offenlegungsschriften 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-Patentschrift 3 869 413 bzw. DE-Offenlegungsschrift 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyäthern (US-Patentschriften 3 383 351, 3 304 273, 3 523 093, 3 110 695; DE-Auslegeschrift 1 152 536) oder Polycarbonatpolyolen (DE-Patentschrift 1 769 795; US-Patentschrift 3 637 909) erhalten werden, sind für das erfindungsgemäße Verfahren geeignet. Bei Verwendung von Polyätherpolyolen, welche gemäß den DE-Offenlegungsschriften 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen,

in welche durch radikalische Pfropfpolymerisation mittels ungesättigter Carbonsäuren sowie gegebenenfalls weiterer olefinisch ungesättigter Monomerer Carboxylgruppen eingeführt wurden (DE-Offenlegungsschriften 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Vertreter der genannten erfindungsgemäß zu verwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z.B. auf den Seiten 45-71, beschrieben. Selbstverständlich können Mischungen der obengenannten Verbindungen mit mindestens zwei gegenüber Isocyanaten reaktionsfähigen Wasserstoffatomen mit einem Molekulargewicht von 400 - 10 000, z.B. Mischungen von Polyäthern und Polyestern, eingesetzt werden.

Von besonderem Vorteil ist es dabei in manchen Fällen, niedrigschmelzende und hochschmelzende Polyhydroxylverbindungen miteinander zu kombinieren (DE-Offenlegungsschrift 2 706 297).

3. Gegebenenfalls als Ausgangskomponenten Verbindungen mit mindestens zwei gegenüber Isocyanaten reaktionsfähigen Wasserstoffatomen und einem Molekulargewicht von 32 bis 400. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf.

Auch in diesem Fall können Mischungen von verschiedenen Verbindungen mit mindestens zwei gegenüber Isocyanaten reaktionsfähigen Wasserstoffatomen mit einem Molekulargewicht von 32 bis 400 verwendet werden.

Als Beispiele für derartige Verbindungen seien genannt:
Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol (US-Patentschrift 3 723 392), Glyzerin, Trimethylolpropan, Hexantriol-(1,2,6), Trimethyloläthan, Pentaerythrit, Chinit, Mannit und Sorbit, Ricinusöl, Diäthylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4'-Dihydroxy-diphenylpropan, Di-hydroxymethyl-hydrochinon,

Äthanolamin, Diäthanolamin, N-Methyldiäthanolamin, Triäthanolamin und 3-Aminopropanol.

Auch Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht (DE-Offenlegungsschrift 2 638 759).

Erfindungsgemaß geeignete aliphatische Diamine sind beispielsweise Äthylendiamin, 1,4-Tetramethylendiamin, 1,11-Undecamethylendiamin, 1,12-Dodecamethylendiamin sowie deren Gemische, 1-Amino-3,3,5-trimethyl-5-aminomethylcyclohexan ("Isophorondiamin"), 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, Diamino-perhydroanthrazene (DE-Offenlegungsschrift 2 638 731 ) und cycloaliphatische Triamine gemäß DE-Offenlegungsschrift 2 614 244. Auch Hydrazin und substituierte Hydrazine, z.B. Methylhydrazin, N,N'-Dimethylhydrazin und deren Homologe sowie Säuredihydrazide kommen erfindungsgemäß in Betracht, z.B. Carbodihydrazid, Oxalsäuredihydrazid, die Dihydrazide von Malonsäure, Bernsteinsäure, Glutarsäure, Adipinsäure, ß-Methyladipinsäure, Sebazinsäure, hydracrylsäure und Terephthalsäure; Semicarbazido-alkylen-hydrazide wie z.B. ß-Semicarbazidopropionsäurehydrazid (DE-Offenlegungsschrift 1 770 591), Semicarbazido-alkylencarbazinester wie z.B. 2-Semicarbazidoäthyl-carbazinester (DE-Offenlegungsschrift 1 918 504) oder auch Amino-semicarbazid-Verbindungen wie z.B. ß-Aminoäthyl-semicarbazido-carbonat (DE-Offenlegungsschrift 1 902 931). Zur Steuerung ihrer Reaktivität können die Aminogruppen ganz oder teilweise durch Aldimin- bzw. Ketimin-Gruppen blockiert sein (US-Patentschrift 3 734 894; DE-Offenlegungsschrift 2 637 115).

Als Beispiele für aromatische Diamine seien Bisanthranilsäureester gemäß den DE-Offenlegungsschriften 2 040 644 und 2 160 590, 3,5- und 2,4-Diaminobenzoesäureester gemäß DE-Offenlegungsschrift 2 025 900, die in den DE-Offenlegungsschriften 1 803 635 (US-Patentschriften 3 681 290 und 3 736 350), 2 040 650 und 2 160 589 beschriebenen estergruppenhaltigen Diamine, die Äthergruppen aufweisenden Diamine gemäß DE-Offenlegungsschriften 1 770 525 und 1 809 172 (US-Patentschriften 3 654 364 und 3 736 295), gegebenenfalls in 5-Stellung substituierte 2-Halogen-1,3-Phenylendiamine (DE-Offenlegungsschriften 2 001 772, 2 025 896 und 2 065 869), 3,3'-Dichlor-4,4'-diamino-diphenylmethan, Toluylendiamin, 4,4'-Diaminodiphenylmethan, 4,4'-Diaminodiphenyldisulfide (DE-Offenlegungsschrift 2 404 976), Diaminodiphenyldithioäther (DE-Offenlegungsschrift 2 509 404), durch Alkylthiogruppen substituierte aromatische Diamine (DE-Offenlegungsschrift 2 638 760), Diaminobenzolphosphonsäureester (DE-Offenlegungsschrift 2 459 491), Sulfonat- oder Carboxylatgruppen enthaltende aromatische Diamine (DE-Offenlegungsschrift 2 720 166) sowie die in der DE-Offenlegungsschrift 2 635 400 aufgeführten hochschmelzenden Diamine genannt. Beispiele für aliphatisch-aromatische Diamine sind die Aminoalkylthioaniline gemäß DE-Offenlegungsschrift 2 734 574.

Als Kettenverlängerungsmittel können erfindungsgemäß auch Verbindungen wie 1-Mercapto-3-aminopropan, gegebenenfalls substituierte Aminosäuren, z.B. Glycin, Alanin, Valin, Serin und Lysin sowie gegebenenfalls

substituierte Dicarbonsäuren, beispielsweise Bernsteinsäure, Adipinsäure, Phthalsäure, 4-Hydroxyphthalsäure und 4-Aminophthalsäure verwendet werden.

Ferner können gegenüber Isocyanaten monofunktionelle Verbindungen in Anteilen von 0,01 bis 10 Gew.-%, bezogen auf Polyurethanfeststoff, als sogenannte Kettenabbrecher mitverwendet werden. Derartige monofunktionelle Verbindungen sind z.B. Monoamine wie Butyl- und Dibutylamin, Octylamin, Stearylamin, N-Methylstearylamin, Pyrrolidin, Piperidin und Cyclohexylamin, Monoalkohole wie Butanol, 2-Äthylhexanol, Octanol, Dodecanol, die verschiedenen Amylalkohole, Cyclohexanol, Äthylenglykolmonoäthyläther.

4. Gegebenenfalls als Hilfs- und Zusatzmittel:

a) Wasser und/oder leicht flüchtige anorganische oder organische Substanzen als Treibmittel. Als organische Treibmittel kommen z.B. Aceton, Äthylacetat, halogensubstituierte Alkane wie Methylenchlorid, Chloroform, Äthylidenchlorid, Vinylidenchlorid, Monofluortrichlormethan, Chlordifluormethan, Dichlordifluormethan, ferner Butan, Hexan, Heptan oder Diäthyläther, als anorganische Treibmittel z.B. Luft, $CO_2$ oder $N_2O$, in Frage. Eine Treibwirkung kann auch durch Zusatz von bei Temperaturen über Raumtemperatur unter Abspaltung von Gasen, beispielsweise von Stickstoff, sich zersetzenden Verbindungen, z.B. Azoverbindungen wie Azodicarbonamid oder Azoisobuttersäurenitril, erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z.B. auf den Seiten 108 und 109, 453 bis 455 und 507 bis 510 beschrieben.

b) Katalysatoren der an sich bekannten Art, z.B. tertiäre Amine, wie Triäthylamin, Tributylamin, N-Methyl-morpholin, N-Äthyl-morpholin, N,N,N',N'-Tetramethyl-äthylendiamin, Pentamethyl-diäthylentriamin und höhere Homologe (DE-Offenlegungsschriften 2 624 527 und 2 624 528), 1,4-Diaza-bicyclo-(2,2,2)-octan, N-Methyl-N'-dimethylaminoäthyl-piperazin, Bis-(dimethylaminoalkyl)-piperazine (DE-Offenlegungsschrift 2 636 787), N,N-Dimethylbenzylamin, N,N-Dimethylcyclohexylamin, N,N-Diäthylbenzylamin, Bis-(N,N-diäthylaminoäthyl)-adipat, N,N,N',N'-Tetramethyl-1,3-butandiamin, N,N-Dimethyl-ß-phenyläthylamin, 1,2-Dimethylimidazol, 2-Methylimidazol, monocyclische und bicyclische Amidine (DE-Offenlegungsschrift 1 720 633), Bis-(dialkylamino)alkyl-äther (US-Patentschrift 3 330 782, DE-Auslegeschrift 1 030 558, DE-Offenlegungsschriften 1 804 361 und 2 618 280) sowie Amidgruppen (vorzugsweise Formamidgruppen) aufweisende tertiäre Amine gemäß den DE-Offenlegungsschriften 2 523 633 und 2 732 292). Als Katalysatoren kommen auch an sich bekannte Mannichbasen aus sekundären Aminen, wie Dimethylamin, und Aldehyden, vorzugsweise Formaldehyd, oder Ketonen wie Aceton, Methyläthylketon oder Cyclohexanon und Phenolen, wie Phenol, Nonylphenol oder Bisphenol, in Frage.

Gegenüber Isocyanatgruppen aktive Wasserstoffatome aufweisende tertiäre Amine als Katalysator sind z.B.

Triäthanolamin, Triisopropanolamin, N-Methyl-diäthanolamin, N-Äthyl-diäthanolamin, N,N-Dimethyl-äthanolamin, deren Umsetzungsprodukte mit Alkylenoxiden wie Propylenoxid und/oder Äthylenoxid sowie sekundär-tertiäre

Amine gemäß DE-Offenlegungsschrift 2 732 292.

Als Katalysatoren kommen ferner Silaamine mit Kohlenstoff-Silizium-Bindungen, wie sie z.B. in der DE-Patentschrift 1 229 290 (entsprechend der US-Patentschrift 3 620 984) beschrieben sind, in Frage, z.B. 2,2,4-Trimethyl-2-silamorpholin und 1,3-Diäthylaminomethyl-tetramethyl-disiloxan.

Als Katalysatoren kommen auch stickstoffhaltige Basen wie Tetraalkylammoniumhydroxide, ferner Alkalihydroxide wie Natriumhydroxid, Alkaliphenolate wie Natriumphenolat oder Alkalialkoholate wie Natriummethylat in Betracht. Auch Hexahydrotriazine können als Katalysatoren eingesetzt werden (DE-Offenlegungsschrift 1 769 043).

Die Reaktion zwischen NCO-Gruppen und Zerewitinoff-aktiven Wasserstoffatomen wird auch durch Lactame und Azalactame stark beschleunigt, wobei sich zunächst ein Assoziat zwischen dem Lactam und der Verbindung mit acidem Wasserstoff ausbildet. Derartige Assoziate und ihre katalytische Wirkung werden in den DE-Offenlegungsschriften 2 062 286, 2 062 289, 2 117 576 (US-Patentschrift 3 758 444), 2 129 196, 2 330 175 und 2 330 211 beschrieben.

Erfindungsgemäß können auch organische Metallverbindungen, insbesondere organische Zinnverbindungen,

als Katalysatoren, verwendet werden. Als organische Zinnverbindungen kommen neben schwefelhaltigen Verbindungen wie Di-n-octyl-zinn-mercaptid (DE-Auslegeschrift 1 769 367; US-Patentschrift 3 645 927) vorzugsweise Zinn(II)-salze von Carbonsäuren wie Zinn(II)-acetat, Zinn(II)-octoat, Zinn(II)-äthylhexoat und Zinn(II)-laurat und die Zinn(IV)-Verbindungen, z.B. Dibutylzinnoxid, Dibutylzinndichlorid, Dibutylzinndiacetat, Dibutylzinndilaurat, Dibutylzinnmaleat oder Dioctylzinndiacetat in Betracht.

Selbstverständlich können alle obengenannten Katalysatoren als Gemische eingesetzt werden. Von besonderem Interesse sind dabei Kombinationen aus organischen Metallverbindungen und Amidinen, Aminopyridinen oder Hydrazinopyridinen (DE-Offenlegungsschriften 2 434 185, 2 601 082 und 2 603 834).

Weitere Vertreter von erfindungsgemäß zu verwendenden Katalysatoren sowie Einzelheiten über die Wirkungsweise der Katalysatoren sind im Kunststoff-Handbuch, Band VII, herausgegeben von Vieweg und Höchtlen, Carl-Hanser-Verlag, München 1966, z.B. auf den Seiten 96 bis 102 beschrieben.

Die Katalysatoren werden in der Regel in einer Menge zwischen etwa 0,001 und 10 Gew.-%, bezogen auf die Gesamtmenge an Verbindungen mit mindestens zwei gegenüber Isocyanaten reaktionsfähigen Wasserstoffatomen, eingesetzt.

c) Oberflächenaktive Zusatzstoffe, wie Emulgatoren und Schaumstabilisatoren. Als Emulgatoren kommen z.B. die Natriumsalze von Ricinusöl-sulfonaten oder Salze von Fettsäuren mit Aminen wie ölsaures Diäthylamin oder stearinsaures Diäthanolamin infrage. Auch Alkali- oder Ammoniumsalze von Sulfonsäuren wie etwa von Dodecylbenzolsulfonsäure oder Dinaphtylmethandisulfonsäure oder von Fettsäuren wie Ricinolsäure oder von polymeren Fettsäuren können als oberflächenaktive Zusatzstoffe mitverwendet werden.

Als Schaumstabilisatoren kommen vor allem Polyäthersiloxane, speziell wasserlösliche Vertreter, infrage. Diese Verbindungen sind im allgemeinen so aufgebaut, daß ein Copolymerisat aus Äthylenoxid und Propylenoxid mit einem Polydimethylsiloxanrest verbunden ist. Derartige Schaumstabilisatoren sind z.B. in den US-Patentschriften 2 834 748, 2 917 480 und 3 629 308 beschrieben. Von besonderem Interesse sind vielfach über Allophanatgruppen verzweigte Polysiloxan-Polyoxyalkylen-Copolymere gemäß DE-Offenlegungsschrift 2 558 523.

d) Reaktionsverzögerer, z.B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und Flammschutzmittel der an sich bekannten Art, z.B. Tris-chloräthylphosphat,

Trikresylphosphat oder Ammoniumphosphat und -polyphosphat, ferner Stabilisatoren gegen Alterungs- und Witterungseinflüsse, Weichmacher und fungistatisch und bakteriostatisch wirkende Substanzen sowie Füllstoffe wie Bariumsulfat, Kieselgur, Ruß oder Schlämmkreide.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z.B. auf den Seiten 103 bis 113 beschrieben.

Durchführung des erfindungsgemäßen Verfahrens:

Die Reaktionskomponenten werden erfindungsgemäß nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der US-Patentschrift 2 764 565 beschrieben werden. Einzelheiten über Verarbeitungseinrichtungen, die auch erfindungsgemäß infrage kommen, werden im Kunststoff-Handbuch, Band VII, herausgegeben von Vieweg und Höchtlen, Carl-Hanser-Verlag,

München 1966, z.B. auf den Seiten 121 bis 205 beschrieben.

Bei der Schaumstoffherstellung kann erfindungsgemäß die Verschäumung auch in geschlossenen Formen durchgeführt werden. Dabei wird das Reaktionsgemisch in eine Form eingetragen. Als Formmaterial kommt Metall, z.B. Aluminium, oder Kunststoff, z.B. Epoxidharz, infrage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Erfindungsgemäß kann man in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter "overcharging" gearbeitet; eine derartige Verfahrensweise ist z.B. aus den US-Patentschriften 3 178 490 und 3 182 104 bekannt.

Bei der Formverschäumung werden vielfach an sich bekannte "äußere Trennmittel", wie Siliconöle, mitverwendet. Man kann aber auch sogenannte "innere Trennmittel", gegebenenfalls im Gemisch mit äußeren Trennmitteln, verwenden, wie sie z.B. aus den DE-Offenlegungsschriften 2 121 670 und 2 307 589 bekanntgeworden sind.

Erfindungsgemäß lassen sich auch kalthärtende Schaumstoffe herstellen (vgl. GB-Patentschrift 1 162 517, DE-Offenlegungsschrift 2 153 086).

Selbstverständlich können aber auch Schaumstoffe durch Blockverschäumung oder nach dem an sich bekannten Doppeltransportbandverfahren hergestellt werden.

Die ausschließliche Umsetzung der erfindungsgemäß zugänglichen Polyhydroxylverbindungen (ohne Mitverwendung anderer gegenüber Isocyanaten reaktiver Komponenten) mit stark elastifizierenden Polyisocyanaten, wie z.B. Polyisocyanaten mit Biuretstruktur (DE-AS 1 543 178) führt zu harten, lichtechten, kratz- und lösungsmittelfesten Beschichtungen und Lacken.

Durch basisch oder sauer katalysierte Propoxylierung und/oder Oxyäthylierung der Polyole lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden. Bezüglich näherer Einzelheiten der Polyätherherstellung sei auf DE-OS 2 639 083 verwiesen.

Durch Umsetzung der erfindungsgemäß hergestellten Gemische aus mehrwertigen Alkoholen mit mehrwertigen Carbonsäuren der oben genannten Art, z.B. Phthalsäure, Isophthalsäure, Terephthalsäure, Tetra- und Hexahydrophthalsäure, Adipinsäure oder Maleinsäure nach den üblichen Verfahren der Polyesterkondensation, wie sie beispielsweise in Houben-Weyl, Methoden der organischen Chemie, Bd. XIV 12, S. 40 beschrieben sind, lassen sich stark verzweigte Polyester synthetisieren, die als Zusätze zu Alkydharzen deren Härte verbessern. Die Hydroxylgruppen enthaltenden Polyester, die aus den erfindungsgemäß hergestellten Hydroxylverbindungen synthetisiert werden, sind selbstverständlich ebenfalls als Ausgangskomponente zur Herstellung von Polyurethankunststoffen brauchbar.

Die erfindungsgemäß hergestellten mehrwertigen Alkohole lassen sich auch sehr leicht mit langkettigen, aliphatischen Monocarbonsäuren, wie Capryl-, Caprin-, Laurin-, Myristin-, Palmitin-, Stearin-, Öl-, Linol-, Arachidon-, oder Behensäure, sowie deren Derivaten, wie z.B. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte der Polyole oder auch Umsetzungsprodukte der erfindungsgemäß zugänglichen Polyhydroxylverbindungen mit langkettigen Monoisocyanaten, wie n-Octyl-, n-Decyl-, n-Dodecyl-, Myristyl-, Cetyl- oder Stearylisocyanat zu Carbamidsäureestern (siehe z.B. K Lindner, Tenside Bd.III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können. Die erfindungsgemäßen Verbindungen lassen sich auch als Feuchthaltemittel in Kosmetika und Kunststoffen verwenden.

Sie können aber z.B. auch als Gefrierschutzmittel dienen oder als Formulierhilfsmittel auf dem Pflanzenschutzsektor.

Die folgenden Beispiele erläutern das erfindungsgemäße Verfahren. Wenn nicht anders vermerkt, sind Mengenangaben als Gewichtsteile bzw. Gewichtsprozente zu verstehen.

Beispiel 1 (Vergleichsbeispiel)

Das Beispiel zeigt, daß bei Verfahren des Standes der Technik bei wesentlich erhöhtem Zeit- und Katalysatoraufwand Produkte mit höherem Anteil an $C_6$-$C_8$-Komponenten erhalten werden als beim erfindungsgemäßen Verfahren.

250 ml einer Formoselösung gemäß Beispiel 1 von DE-OS 2 721 186 werden zusammen mit 80 g Raney-Nickel in einem 0,7 l-Autoklaven bei 150 bar Wasserstoffdruck 4 Stunden bei 30°C, dann 1 Stunde bei 60°C und schließlich 1 Stunde bei 100°C hydriert.

Man erhält eine leicht gelbliche Lösung von Polyhydroxylverbindungen mit einem Gehalt an reduzierenden Gruppen von 0,018 % und der folgenden molekularen Verteilung:

| | |
|---|---|
| Verbindungen mit 2 C-Atomen | 0,8 |
| Verbindungen mit 3 C-Atomen | 2,2 |
| Verbindungen mit 4 C-Atomen | 5,6 |
| Verbindungen mit 5 C-Atomen | 30,4 |
| Verbindungen mit 6 C-Atomen | 40,0 |
| Verbindungen mit 7 C-Atomen und mehr | 21,0 |

Beispiel 2 (Vergleichsbeispiel)

In einem 3 l-Edelstahlautoklaven werden 100 g Katalysator (Raney-Ni/Fe im Verhältnis 85:15), suspendiert in 1 l Wasser, vorgelegt und auf Hydriertemperatur

(150°C) aufgeheizt. Dann wird das überstehende Volumen mit Wasserstoffgas bis zum Betriebsdruck von 150 bar gefüllt. 500 ml einer gemäß Beispiel 1 von DE-OS 2 721 186 hergestellten 50 %igen Formoselösung mit einem Gehalt an redzierbaren Gruppen (bestimmt als Carbonylgruppen) von 11,1 % werden mit NaOH auf einen pH-Wert von 10,0 eingestellt und im Verlauf von 6 min. in den Autoklaven eingepumpt. Anschließend wird 6 min. nachhydriert. Dann läßt man 500 ml der hydrierten Lösung über ein Steigrohr mit Fritte, welche den Katalysator zurückhält, ab, pumpt die nächste Charge zu und hydriert sie wie die erste. Mit 160 weiteren Chargen von je 500 ml wird in gleicher Weise verfahren. Ein Katalysatorverlust ist nach dieser Zyklenzahl nicht festzustellen.

Die hydrierten Lösungen werden gesammelt, über Ionenaustauscher entsalzt und im Dünnschichtverdampfer von der Hauptmenge an Wasser befreit. Man erhält einen gelb gefärbten Formit mit folgenden Eigenschaften:

| | |
|---|---|
| Rest-Wassergehalt: | 1,1 % |
| Rest-Carboxylgehalt: | 0,016 % |
| OH-Zahl: | 1390 |

Komponentenverteilung:

| | | |
|---|---|---|
| Verbindungen mit | 2 C-Atomen: | 7,9 % |
| | 3 C-Atomen: | 22,0 % |
| | 4 C-Atomen: | 19,0 % |
| | 5 C-Atomen: | 19,1 % |
| | 6 C-Atomen: | 21,0 % |
| | 7 und mehr C-Atomen: | 11,0 % |

Beispiel 3

In einem 3 l-Edelstahlautoklaven werden 100 g Katalysator (Raney-Ni/Fe im Verhältnis 85/15), suspendiert in 1 l Wasser, vorgelegt und auf die Hydriertemperatur von 150°C aufgeheizt. Dann wird das überstehende Volumen mit Wasserstoffgas bis zum Betriebsdruck von 150 bar gefüllt. 500 ml der gemäß Beispiel 2 erhaltenen, nicht eingeengten 50 %igen Formitlösung werden über einen in der H-Form vorliegenden handelsüblichen Kationenaustauscher (sulfoniertes, mit Divinylbenzolvernetztes Polystyrol) geleitet, wobei sich ein pH-Wert von 3,2 einstellt, und 30 Minuten lang bei 30 °C stehengelassen. Anschließend wird der pH-Wert der Lösung durch Überleiten über einen in der OH-Form vorliegenden handelsüblichen Anionenaustauscher und Zugabe von Natriumhydroxid wieder auf 10,0 eingestellt. Die alkalisch gemachte Lösung wird innerhalb von 8 Min. in den Autoklaven gepumpt und 10 Min. nachhydriert. Man läßt danach 500 ml der Lösung über ein Steigrohr mit Fritte, welche den Katalysator zurückhält, ab. Mit 140 weiteren Chargen wird in gleicher Weise verfahren. Ein Katalysatorverlust kann dabei nicht festgestellt werden.

Die hydrierten Lösungen werden gesammelt, über Ionenaustauscher entsalzt und im Dünnschichtverdampfer von Wasser befreit. Man erhält einen glasklaren, farblosen Formit folgender Zusammensetzung:

| | | |
|---|---|---|
| Verbindungen mit | 2 C-Atomen : | 7,5 % |
| | 3 C-Atomen : | 22,3 % |
| | 4 C-Atomen : | 21,8 % |

Verbindungen mit 5 C-Atomen : 17,2 %
6 C-Atomen : 20,1 %
7 und mehr C-Atomen : 11,1 %

Beispiel 4

Herstellung eines Polyurethanschaumstoffs

25 Teile eines auf Äthylendiamin gestarteten Polypropylenoxids (OH-Zahl: 470),
22 Teile des Formits aus Beispiel 3,
10 Teile Trichloräthylphosphat,
15 Teile Monofluortrichlormethan,
0,5 Teile Dimethylbenzylamin
0,5 Teile eines handelsüblichen Siliconstabilisators (L-5340 der UCC)
und
75 Teile eines technischen Phosgenierungsproduktes von Anilin/Formaldehyd-Kondensaten (NCO-Gehalt: 29 %)

werden intensiv vermischt und das Gemisch in einer offenen Form aufschäumen gelassen.

Man erhält einen harten, feinzelligen Schaumstoff mit großer Reißfestigkeit und Dimensionsstabilität.

Patentansprüche

1. Verfahren zur Herstellung von niedermolekularen, mehrwertigen Alkoholen durch Hydrierung von Formoselösungen in Gegenwart von Metallkatalysatoren, dadurch gekennzeichnet, daß man

   A) in einer ersten Stufe eine mindestens 20 %ige Lösung des zu reduzierenden Produkts, dessen pH-Wert vor der Hydrierungsreaktion auf einen Wert zwischen 7,5 und 12,5 eingestellt wurde, chargenweise in einen Reaktor einbringt, so daß der Gehalt an reduzierbaren Gruppen (bestimmt als Carbonylgruppen) innerhalb des Reaktors einen Wert von 2 Gew.-% nicht überschreitet, und chargenweise in Gegenwart einer Gesamtmenge an Katalysator von $10^{-4}$ bis $5.10^{-2}$ Gew.-%, bezogen auf Gesamtmenge an zu reduzierendem Ausgangsprodukt, hydriert, wobei der Katalysator stationär im Reaktor verbleibt, danach das Reaktionsprodukt der ersten Hydrierstufe chargenweise aus dem Reaktor abzieht, nachdem der Gehalt an reduzierbaren Gruppen (bestimmt als Carbonylgruppen) unter 1,5 Gew.-% gesunken ist, den pH-Wert des so vorhydrierten Produkts auf 1 bis 6 einstellt und

   B) in einer zweiten Stufe das Reaktionsprodukt der ersten Stufe chargenweise in einen zweiten Reaktor einbringt, wobei der pH-Wert des Produkts unmittelbar vor dem Einbringen in den zweiten Reaktor wieder auf 7,5 bis 12,5 eingestellt wird, chargenweise in Gegenwart einer Gesamtmenge an

Katalysator von $10^{-4}$ bis $5.10^{-2}$ Gew.-%, bezogen auf Gesamtmenge an in der ersten Stufe vorhydriertem Produkt, hydriert, wobei der Katalysator stationär im Reaktor verbleibt, und nach einer Verweilzeit von 5 Min. bis 4 Stunden das Reaktionsprodukt chargenweise aus dem Reaktor abzieht.

2. Verfahren nach Anspruch 1, dadurch gekennzeichnet, daß jede Charge der ersten bzw. der zweiten Stufe die 3 bis 30 fache Menge des im ersten bzw. im zweiten Reaktor vorhandenen Katalysators beträgt.

3. Verfahren nach Ansprüchen 1 und 2, dadurch gekennzeichnet, daß die einzelnen Chargen mit einer Geschwindigkeit zugepumpt werden, welche der Füllung von 1/6 des Reaktorvolumens in 3 bis 120 Minuten entspricht.

4. Verfahren nach Anspruch 1 bis 3, dadurch gekennzeichnet, daß nach dem Zupumpen jeder Charge der ersten Stufe während einer Zeitdauer, die der halben bis 4-fachen Zupumpzeit entspricht, nachhydriert wird und daß nach dem Zupumpen jede Charge der zweiten Stufe während einer Zeitdauer, die der halben bis 5-fachen Zupumpzeit entspricht, nachhydriert wird.

5. Verfahren nach Ansprüchen 1 bis 4, dadurch gekennzeichnet, daß das zu hydrierende Ausgangsprodukt neben Formose bis zu 80 Gew.-% (bezogen auf Gesamtmenge an zu hydrierendem Produkt) an weiteren natürlichen und/oder synthetischen Zuckern enthält.

6. Verfahren nach Ansprüchen 1 bis 5, dadurch gekennzeichnet, daß für beide Hydrierstufen Nickel-haltige Katalysatoren eingesetzt werden.

7. Verfahren nach Ansprüchen 1 bis 6, dadurch gekennzeichnet, daß als Katalysator Raney-Nickel, welches gegebenenfalls mit Eisen und/oder Zink modifiziert ist, eingesetzt wird.

8. Verwendung der nach Ansprüchen 1 bis 7 hergestellten Alkoholgemische als Startkomponente für die Herstellung vpn Polyäther- und/oder Polyesterpolyolen.

9. Verwendung der nach Ansprüchen 1 bis 7 hergestellten Alkoholgemische zur Herstellung von nichtionogenen, oberflächenaktiven Verbindungen durch Umsetzung mit Fettsäuren, Fettsäurederivaten, langkettigen aliphatischen Monoisocyanaten oder durch partielle Oxäthylierung.

10. Verfahren zur Herstellung von gegebenenfalls zellförmigen Polyurethankunststoffen durch Umsetzung von

A) Polyisocyanaten mit
B) mindenstens zwei aktive Wasserstoffatome aufweisenden Verbindungen mit einem Molekulargewicht zwischen 32 und 400, gegebenenfalls

C) mindestens zwei aktive Wasserstoffatome aufweisenden Verbindungen mit einem Molekulargewicht zwischen 400 und 10 000 sowie gegebenenfalls

0007100



D) Treibmitteln, Katalysatoren und weiteren an sich bekannten Zusatzstoffen,

dadurch gekennzeichnet, daß als Komponente B), gegebenenfalls nur anteilsweise, gemäß Anspruch 1 bis 7 hergestellte Gemische niedermolekularer, mehrwertiger Alkohole eingesetzt werden.